(19) Europäisches Patentamt
European Patent Office
Office européen des brevets

(11) EP 2 615 080 A1

(12) EUROPEAN PATENT APPLICATION

(43) Date of publication:
**17.07.2013 Bulletin 2013/29**

(21) Application number: **12150892.3**

(22) Date of filing: **12.01.2012**

(51) Int Cl.:
*C07C 211/27* (2006.01)
*C07C 239/20* (2006.01)
*C07C 227/34* (2006.01)
*C07C 303/40* (2006.01)
*C07C 231/06* (2006.01)
*C07F 5/02* (2006.01)
*C07C 269/06* (2006.01)
*C07C 67/31* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **LEK Pharmaceuticals d.d.
1526 Ljubljana (SI)**

(72) Inventor: **The designation of the inventor has not yet been filed**

(74) Representative: **Kunic Tesovic, Barbara
Lek Pharmaceuticals d.d.
Sandoz Development Center Slovenia - Patents
Verovskova 57
1526 Ljubljana (SI)**

# (54) Preparation of Optically Pure ß-Amino Acid Type Active Pharmaceutical Ingredients and Intermediates thereof

(57) The present invention relates to the preparation of optically resolved chiral compounds of β-amino acid type active pharmaceutical ingredients (API), more specifically to β-aminobutyryl substituted compounds and especially β-aminobutyryl compounds having γ-bound aryl groups. The present invention more particularly relates to the preparation of enantiomerically enriched chiral compounds useful as intermediates for the preparation of anti-diabetic agents, preferably sitagliptin.

EP 2 615 080 A1

**Description**

Field of the Invention

The present invention relates to the preparation of optically resolved chiral compounds of β-amino acid type active pharmaceutical ingredients (API), more specifically to β-aminobutyryl substituted compounds and especially β-aminobutyryl compounds having γ-bound aryl groups. The present invention more particularly relates to the preparation of enantiomerically enriched chiral compounds useful as intermediates for the preparation of anti-diabetic agents, preferably sitagliptin.

Background of the Invention

β-Amino acids are of interest in the preparation of active pharmaceutical ingredients (APIs). The β-amino acid moiety in APIs of interest is normally part of a complex whole structure. Complexity is typically enhanced when considering a chiral center at the β-position of the β-aminobutyryl group and the general desire to obtain enantiopure compounds.

A particularly interesting class of APIs having β-amino acid structural moieties are dipeptidyl peptidase-4 (DPP-4) inhibitors which act as antidiabetic agents. DPP-4 inhibitors are oral antidiabetic drugs, which reduce glucose blood levels by a new mechanism of action in which the DPP-4 inhibitors ("gliptins") inhibit inactivation of glucagon-like peptide (GLP), which stimulates insulin secretion. Gliptins can be used for treatment of diabetes mellitus type 2 either alone or in combination with other oral antihyperglycemic agents, such as metformin or thiazolidinediones.

Sitagliptin (CAS Registry Number 486460-32-6, IUPAC Name: (*R*)-4-oxo-4-[3-(trifluoromethyl)-5,6-dihydro [1,2,4]triazolo[4,3-*a*]pyrazin-7(8*H*)-yl]-1-(2,4,5-trifluorophenyl)butan-2-amine) is an anti-diabetic agent and a potent inhibitor of the DPP-IV. It is represented by the structure:

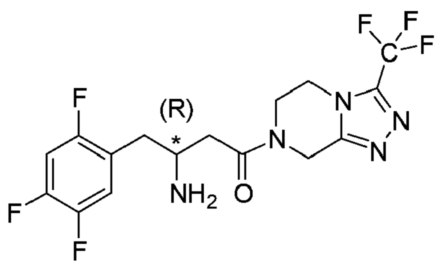

However, obtainment of a desired enantiomerically pure form at the chiral center of β-aminobutyryl substituted compounds and especially β-aminobutyryl compounds having γ-bound aryl groups remains a permanent challenge, especially for industrial production.

This can be adequately demonstrated by referring for example to conventional synthetic schemes directed to obtaining enantiomerically pure forms of the representative compound sitagliptin or intermediates thereof. To this end, in order to achieve optically pure sitagliptin molecule, several approaches have been presented up to now. Usually chirality was introduced mostly by use of transition metal catalysts (Rh, Ru) in a combination with chiral ligands (WO 03/004498; WO 09/064476; WO 04/085378; WO 05/097733; WO 06/081151; Hansen K. B.; et. al. Org. Process Res. Dev. 2005, 9, 634-639; Hansen, K. B.; et. al. J. Am. Chem. Soc. 2009, 131, 8798-8804) or using organic chiral auxiliaries like chiral dihydropyrazine derivatives, chiral epoxides etc. (WO 03/004498). Another approach is described in WO 2011/102640 A2, where an already previously enantiomerically enriched sample of 3-azido-4-(2,4,5-trifluorophenyl) butanoic acid is reacted with an amine to obtain the amine salt of the corresponding carboxylic acid- and to finally obtain sitagliptin with high compound purity and optical purity. Amines of general structure $NR_1,R_2,R_3$, which may be a primary, secondary or tertiary amine, are encompassed to react with the β-azido acid intermediate, wherein each of $R_1$, $R_2$ and $R_3$ may be independently selected from H, $C_1$-$C_5$-alkyl, benzyl, 1-phenylethyl, or 2,2-diphenylethyl; or linked together with the adjacent nitrogen atom to form a pyridine, piperidine, morpholine, pyrrolidine, or piperazine ring. Various achiral amines and one chiral amine, methylbenzylamine, are specifically mentioned.

Object of the Invention

It was therefore an object of the present invention to provide an improved preparation of enantiomerically pure β-amino acids suitable for industrial production specially to provide new diastereomeric amine salts suitable for formation of optically pure β-amino acid intermediates useful in preparation of anti-diabetic agents, preferably sitagliptin.

Summary of the Invention

The present invention provides a process for preparing enantiomerically resolved compound of formula I

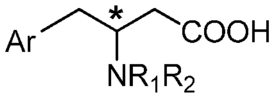

I

wherein the stereogenic center marked by * is in (*R*) or in (*S*) configuration in enantiomerically resolved state, wherein $R_1$ and $R_2$ are identical or different, and are independently selected from

(i) hydrogen;
(ii) alkyl residues optionally chiral, having from 1 to 12 carbon atoms, wherein the alkyl residues are optionally aryl and/or aryloxy substituted;
(iii) alkyloxy residues optionally chiral, having from 1 to 12 carbon atoms, wherein the alkyloxy residues are optionally aryl substituted;
(iv) aryl residues optionally chiral, having from 6 to 24 carbon atoms, wherein the aryl residues are optionally alkyl and/or alkyloxy substituted;
(v) aryloxy residues optionally chiral, having from 6 to 24 carbon atoms, wherein the aryloxy residues are optionally alkyl substituted;
(vi) benzyl;
(vii) alkaloyl residues optionally chiral, having from 2 to 13 carbon atoms, wherein the alkaloyl residues are optionally aryl substituted;
(viii) aroyl residues optionally chiral, having from 7 to 25 carbon atoms, wherein the aryloxy residues are optionally alkyl substituted; and
(ix) alkoxycarbonyl residues optionally chiral, having from 2 to 13 carbon atoms;
(x) aryloxycarbonyl residues optionally chiral, having from 7 to 25 carbon atoms;
(xi) tosyl;
(xii) silyl residues optionally chiral, having from 3 to 15 carbon atoms; and
(xiii) silyloxy residues optionally chiral, having from 3 to 15 carbon atoms; and Ar denotes respectively unsubstituted or substituted phenyl, alkyl-aryl, alkoxy-aryl, the process comprising the steps of:

(a) providing an enantiomeric mixture of the compound I

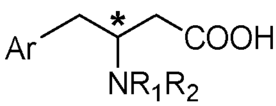

**I**

wherein $R_1$, $R_2$ and Ar are as defined above, and wherein * indicates a stereogenic center, present in an enantiomeric mixture;
(b) forming a diastereomeric salt with an amine $NR_3R_4R_5$ to obtain a compound of formula II, wherein $R_1$, $R_2$ and Ar are as defined above and $R_3$, $R_4$ and $R_5$ are independently selected from residues to form a chiral amine, wherein two or all three among $R_3$, $R_4$ and $R_5$ may together form a ring structure, provided that at least one of $R_3$, $R_4$ and $R_5$ or said ring structure contains a chiral center.

Preferably $R_3$, $R_4$ and $R_5$ are selected from the group of hydrogen, linear or branched $C_1$-$C_{10}$-alkyl, substituted $C_1$-$C_{10}$-alkyl, preferably aryl, heteroaryl, or hydroxy substituted $C_1$-$C_{10}$-alkyl or two or all three among $R_3$, $R_4$ and $R_5$

form a ring structure such as piperidine, pyolidine, piperazine, morpholine, quinuclidine, wherein at least one of the substituents $R_3$, $R_4$ and $R_5$ or said ring structure contains at least one chiral center, preferably the chiral substituent is selected from α-aryl substituted alkyl, such as 1-phenylethyl, 1-phenyl-propyl, 1-naphthylethyl or the amine $NR_3R_4R_5$ represents an easy available chiral natural structure, preferably selected from alkaloids. The most preferred amines are selected from (1-phenylethyl)amine, *N*-benzyl-*N*-(1-phenylethyl)amine, *N*,*N*-bis[α-methylbenzyl]amine, 1-phenyl-propylamine, (1-naphthylethyl)amine, *N*-(2-methoxybenzyl)-*N*-1-phenylethyl, N-(3,4-dimethoxybenzyl)-*N*-(1-phenylethyl)-amine, phenylglicinol or from alkaloids such as cinchonidine, cinchonine, quinidine, quinine, ephedrine, brucine, nicotine, spartein.

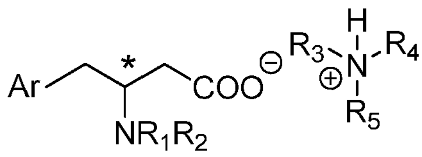

**II**

providing optical resolution of the diastereomeric salt of formula II into a desired diastereomerically resolved salt form; and

(c) converting the optically resolved diastereomeric salt of formula II into the corresponding enantiomerically resolved compound of formula I.

The present invention further provides, as a useful intermediate compound, a diastereomeric salt of formula II

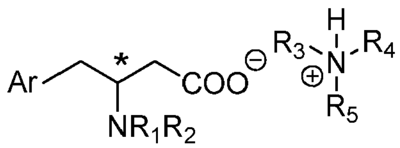

**II**

wherein * indicates a stereogenic center,
$R_1$ and $R_2$ are identical or different, and are independently selected from

(i) hydrogen;
(ii) alkyl residues optionally chiral, having from 1 to 12 carbon atoms, wherein the alkyl residues are optionally aryl and/or aryloxy substituted;
(iii) alkyloxy residues optionally chiral, having from 1 to 12 carbon atoms, wherein the alkyloxy residues are optionally aryl substituted;
(iv) aryl residues optionally chiral, having from 6 to 24 carbon atoms, wherein the aryl residues are optionally alkyl and/or alkyloxy substituted;
(v) aryloxy residues optionally chiral, having from 6 to 24 carbon atoms, wherein the aryloxy residues are optionally alkyl substituted;
(vi) benzyl;
(vii) alkaloyl residues optionally chiral, having from 2 to 13 carbon atoms, wherein the alkaloyl residues are optionally aryl substituted;
(viii) aroyl residues optionally chiral, having from 7 to 25 carbon atoms, wherein the aryloxy residues are optionally alkyl substituted; and
(ix) alkoxycarbonyl residues optionally chiral, having from 2 to 13 carbon atoms;
(x) aryloxycarbonyl residues optionally chiral, having from 7 to 25 carbon atoms;
(xi) tosyl;
(xii) silyl residues optionally chiral, having from 3 to 15 carbon atoms;
(xiii) silyloxy residues optionally chiral, having from 3 to 15 carbon atoms;

Ar denotes respectively unsubstituted or substituted phenyl, alkyl-aryl, alkoxy-aryl; and wherein $NR_3R_4R_5$ represents an amine and $R_3$, $R_4$ and $R_5$ are independently selected from residues to form a chiral amine, wherein two or all three among $R_3$, $R_4$ and $R_5$ may together form a ring structure, provided that at least one of $R_3$, $R_4$ and $R_5$ or said ring structure contains a chiral center as described above.

It was found that a chiral resolution approach via diastereoisomeric salt formation is highly amenable to industrial production when applied to an enantiomeric mixture of the compound of formula I. Although - besides other techniques such as stereospecific/asymmetric chemical synthesis schemes, and especially besides other chiral resolution techniques such as assistance of chiral auxiliaries, and achiral or chiral chromatography - chiral resolution approaches via diastereoisomeric salt formation is generally known as indicated in the general Scheme 1 below, it is neither known or foreseeable in advance at which step and with which intermediate compound (i.e. the specific compound as the substrate of salt formation), a diastereomeric salt formation may be successful with which salt type, and whether and how resolution may work and which appropriate conditions, including the question whether fractional crystallization will lead to an appropriate diastereomeric excess. It was however found that when the compound of formula I is treated with chiral amine, two diastereoisomeric salts with different solubility can be efficiently formed which can be readily separated with crystallization, notably by fractional crystallization, and that this can be done at an effective step and with a useful intermediate of whole synthesis schemes to eventually obtain active pharmaceutical ingredients amenable to a basic gliptin structural configuration, more specifically of sitagliptin and related compounds. Distinct from several conventional methods described how to introduce chirality into the molecule of sitagliptin, intermediates thereof or related compounds, the process according to the present invention does not suffer from drawbacks of using expensive, hazardous and also toxic chiral transition metal inductors. Moreover, the undesirable enantiomer may be recycled thereby allowing compensation of material loss by regaining a second and optionally further cycle(s) of chiral resolution(s) via diastereoisomeric salt and thereby allowing a substantial increase in the overall yield.

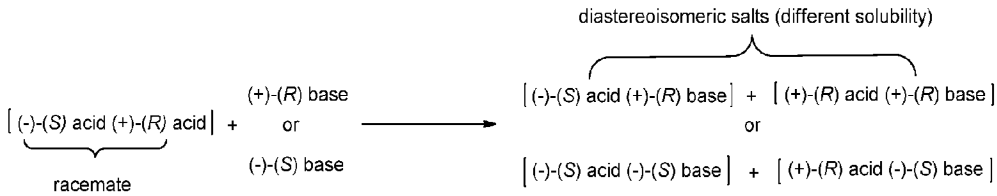


**Scheme 1**

The term "enantiomeric excess" or the most often expressed "percent of enantiomeric excess" is defined herein as usually understood by the common formula ee = $|(R - S) / (R + S)|$ x 100 wherein "*R*" and "*S*" denote the respective amounts of enantiomeric forms of the compound of interest such that $R + S = 1$.

The term "optically enriched" means herein an enantiomeric purity of at least 60%, preferably of at least 80%.

The term "optically pure" means herein an enantiomeric purity of at least 98%. Likewise the term "diastereomeric excess" is defined herein as usually understood by the common formula

$$de = |(1^{st}\text{ dia-form} - (\text{sum of other dia-forms}) / (\text{total sum of all dia-forms})| \times 100,$$

wherein "$1^{st}$ dia-form" denotes the diastereomeric form of interest as opposed to other existing diastereomeric forms of the corresponding salt (see Scheme 1 for illustration).

<u>Description of Preferred Embodiments</u>

The present invention will now be described in further detail with respect to preferred embodiments and specific examples, which embodiments and examples however are only illustrative to explain the invention without however limiting the invention thereto.

The invention presents preparation of diastereoisomeric amine salts useful and specifically designed for efficient, reliable and industrially applicable optical resolution of β-amino acid type active pharmaceutical ingredients, notably

having as structural moiety β-aminobutyryl with γ-bound aryl groups. In particular, diastereomeric amine salts of β-amino- and γ-aryl- substituted butanoic acid (in which the β-amino- and γ-aryl-groups may be desirably substituted depending on the compound of interest) are formed, which proved to be useful for formation of optically pure β-amino acid intermediate in the synthesis process of typical gliptin compounds such as sitagliptin. After separation, the resulting optically pure amine salts can be easily converted back to the free acid in order to liberate the desired enantiomer.

Advantageously, it has been found that representative compounds of formula I, as demonstrated by intermediate compounds suitable for the synthesis of sitagliptin, the enantiomerically resolved compound of formula I is obtained enantiomerically enriched with excess (ee) of at least 60%, preferably of at least 80%, and is even obtainable enantiomerically pure, optionally by additional recrystallizations of the salt of formula II.

The present invention surprisingly offers an economical approach in sitagliptin process in contrast to very expensive asymmetric prior art approaches, where transition metal catalysts (in some cases even toxic metals) in combination with expensive chiral ligands or even special enzymes are usually used for asymmetric induction to enable appropriate optical purity of the desired material.

In the process of the present invention, the enantiomeric mixture provided in step (a) typically is a racemate of both (*R*)- and (*S*)-configurations of the compound of formula I. Such racemate is usually obtained in non-stereoselective preparations or symmetric synthesis of the compound of formula I.

There are in principle various synthetic possibilities to prepare and thereby provide the enantiomeric mixture of the compound of formula I to be used in step (a)

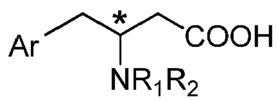

I

For example and in a preferred embodiment, such prior process comprises the steps of:

(0-1) providing a compound of formula IV,

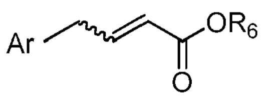

IV

wherein $R_6$ is selected from alkyl residues having from 1 to 6 carbon atoms;

(0-2) reacting the compound of formula IV with an amine of formula $HNR_1R_2$, wherein $R_1$ and $R_2$ are as defined above to be later present in the compound of formula I, in a protic solvent, particularly in water; or in a mixture of protic solvents, wherein the mixture particularly comprises water; or without adding of solvents in step (0-2); and

(0-3) cleaving the ester group to obtain the compound of formula I.

The solvent in step (0-2) may be selected from water, methanol, ethanol, *iso*-propanol, *tert*-butanol, trifluoroethanol, hexafluoro-2-propanol, amyl alcohol and any combination thereof, and is particularly water. It is preferred that step (0-2) is carried out without adding solvents. This step (0-2), which may be a non-catalyzed reaction, an acid-catalyzed reaction (preferably using a Lewis acid or a Brønsted acid), a transition metal catalyzed reaction, or an organocatalyzed reaction, may be carried out at a temperature of 20 °C to 100 °C, preferably of 20 °C to 85 °C. Preferably, step (0-2) is carried out in the presence of a promoter, in particular a substance selected from fluorinated alcohols, in particular selected from trifluoroethanol, hexafluoro-2-propanol, and any combination thereof, and/or step (0-2) is carried out in the presence of a surfactant, in particular wherein the surfactant is selected from ionic, nonionic surfactants, and the combination thereof.

Alternatively, and according to another preferred embodiment, such prior process comprises the steps of:

(0-1') providing a compound of formula IV

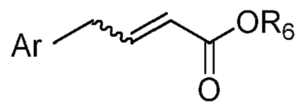

IV

wherein $R_6$ is selected from alkyl residues having from 1 to 6 carbon atoms;
(0-2') reacting the compound of formula IV with a borating agent in a suitable solvent to obtain an intermediate of formula V,

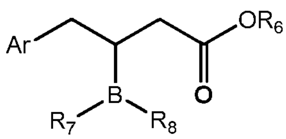

V

wherein * denotes a stereogenic center, and $R_6$ is selected from alkyl residues having from 1 to 6 carbon atoms; wherein $R_7$ and $R_8$ are identical or different, and are selected from

(i) alkyl or alkoxy residues, each having from 1 to 12 carbon atoms, wherein each alkyl or alkoxy residue is optionally aryl substituted,
(ii) aryl or aryloxy residues, each having from 6 to 14 carbon atoms, wherein each aryl or aryloxy residue is optionally alkyl substituted;
(iii) halides; and
(iv) wherein $R_7$ and $R_8$ optionally form a chiral or non-chiral 5 to 10, particularly 5 to 6, membered mono or bicyclic ring, wherein the ring is optionally substituted at least one position with an alkyl residue having from 1 to 12 carbon atoms and/or an aryl residue having from 6 to 14 carbon atoms, and wherein $R_7$ and $R_8$ optionally form an *O*-benzenedioxy residue; and

(0-3') converting the compound of formula V to the compound of formula I.

Step (0-1') can be accomplished as a transition metal catalysed process, using a catalyst comprising a transition metal compound, an base and at least one ligand, preferably wherein the transition metal compound is selected from copper(I) chloride, copper(II) bromide, copper(I) oxide, copper(II) oxide, copper(I) acetate, copper(II) triflate, copper(II) carbonate, and any combination thereof and particularly wherein the base is selected from NaO*t*Bu, KO*t*Bu, $K_2CO_3$, $Na_2CO_3$, KOAc, NaOAc, and any combination thereof, more particularly NaO*t*Bu. Typically, when the transition metal compound is copper(II) carbonate the transition metal catalyzed process is carried out in the absence of a base. According to another step (0-1') can be achieved also without a transition metal catalyst but using a base and at least one ligand, wherein the base is preferably selected from cesium carbonate, cesium chloride, cesium fluoride, cesium iodide, and any combination thereof, and is particularly cesium carbonate.

In step (0-3'), the compound of formula V may be converted to the compound of formula I by an amination process, for example an animation process comprising the sub-steps of: reacting the compound of formula V with an organo-zinc compound and/or an organo-magnesium compound in a suitable solvent, and reacting with an electrophilic aminating reagent in a suitable solvent; or an animation process comprising the sub-steps of: reacting the compound of formula V with a halo-boron agent and/or a bifluoride agent, and reacting with an azide aminating reagent.

Alternatively step (0-3') comprises the steps of: reacting the compound of formula V with an oxidation agent in a suitable solvent to obtain a compound of formula VI

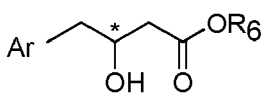

VI

wherein * denotes the stereogenic center, and wherein $R_6$ is as defined above; and reacting the intermediate of formula VI with an aminating agent to obtain an intermediate of formula I. The aforementioned oxidation agent may be selected from the group consisting of sodium perborate hydrate, hydrogen peroxide, sodium hypochlorite, sodium percarbonate, sodium bromate, potassium bromate, sodium chlorate, potassium chlorate, oxone, and any combination thereof.

According to a preferred embodiment, in the process according to the present invention the structural moiety of $NR_1R_2$ is selected from the group consisting of

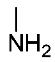

Ia

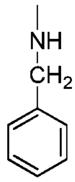

Ib

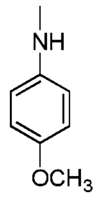

Ic

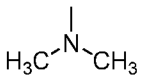

Id

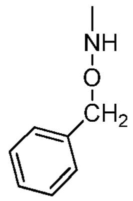

Ie

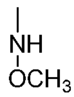

If

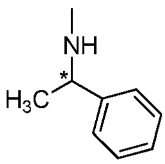

Ig

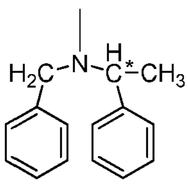

Ih

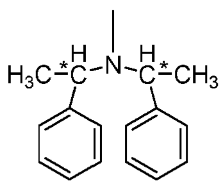

Ii

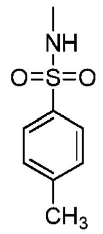

Ij

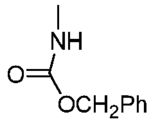

Ik

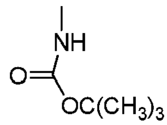

Il

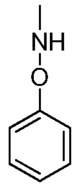

Im

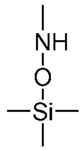

In

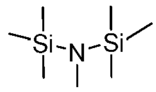

Io

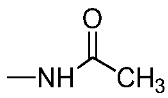

Ip

In the above description, Ar may be preferably selected from, without be limited to, phenyl or substituted phenyl. Preferably, Ar is substituted phenyl defined of the formula

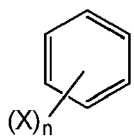

wherein X is halogen selected from fluoro, chloro, or bromo, preferably fluoro, same or different, and n is 1-4. In particular, Ar is substituted phenyl defined by the formula

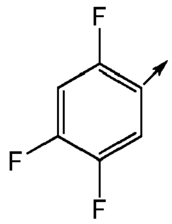

wherein the arrow indicates the binding site of the Ar group to the gama(γ)-carbon atom of the β-amino acid structural moiety in the compounds of formula I and II.

Particularly preferred compounds of formula I, to which the chiral resolution approach via diastereoisomeric salt formation is advantageously applied, are selected from the group consisting of the following compound Ia' to Ip':

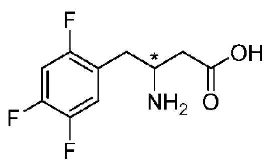

Ia'

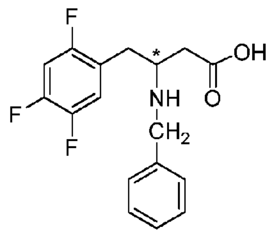

Ib'

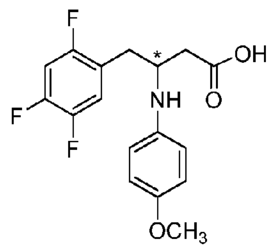

Ic'

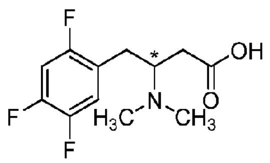

Id'

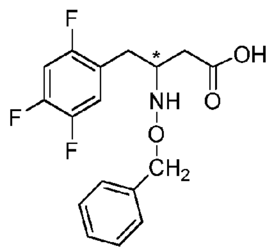

Ie'

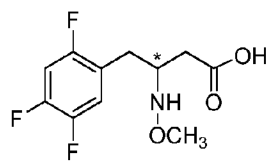

If'

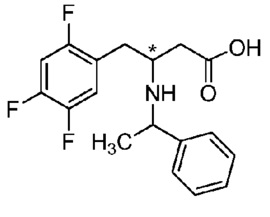

Ig'

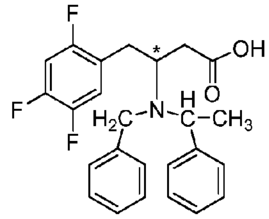

Ih'

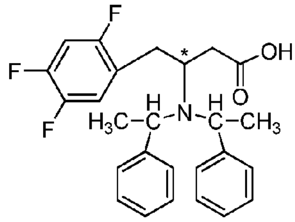

Ii'

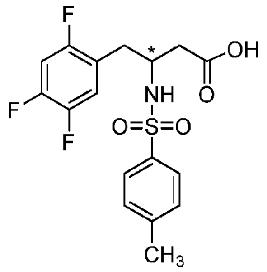

Ij'

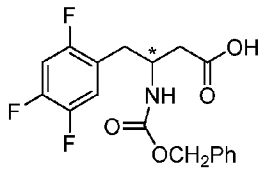

Ik'

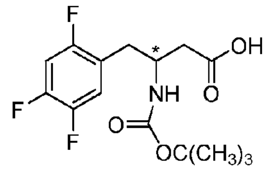

Il'

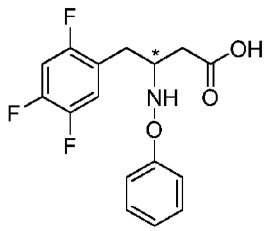

Im'

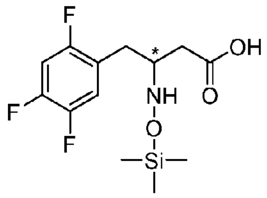

In'

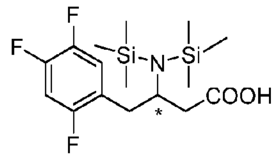

Io'

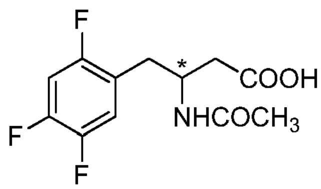

Ip'

In order to form the diastereomeric salt of the enantiomeric mixture of the compound of formula I, chiral amines $NR_3R_4R_5$, wherein the substituents $R_3$, $R_4$ and $R_5$ are the same or different and at least one of them is a chiral moiety, are selected. In one preferred option the chiral substituent is selected from $\alpha$-aryl substituted alkyl, such as 1-phenylethyl, 1-phenyl-propyl, 1-naphthylethyl, 1-phenylethyl, while the achiral substituent is selected from hydrogen, alkyl or benzyl. Examples of suitable chiral amines to form the diastereomeric salt of the enantiomeric mixture of the compound of formula I include, without being limited to, 1-phenylethyl amine (A), *N*-benzyl-1-phenylethyl amine (B), *N*,*N*-bis[$\alpha$-methylbenzyl] amine (C), 1-phenyl-propyl amine (D), (1-naphthylethyl) amine (E), 1-(3-methoxyphenyl)ethyl amine (F), 1-(3,4-dimethoxy-phenyl)ethyl amine (G), *N*-methylbutan-3-ol-2-amine

(H), 2-phenylglicinol (I), respectively present in *(R)*- and/or *(S)*-configurations, wherein stereocenter is marked by *.

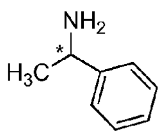

(A)

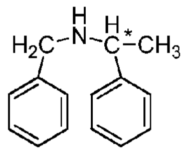

(B)

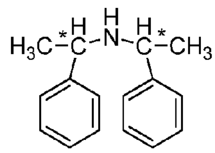

(C)

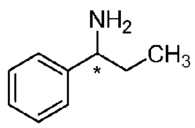

(D)

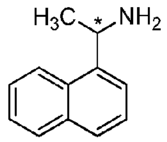

(E)

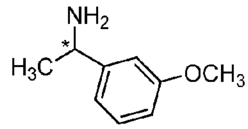

(F)

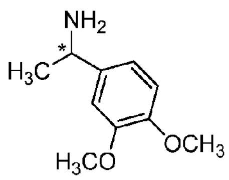

(G)

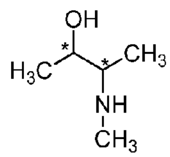

(H)

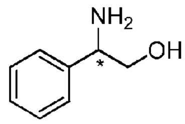

(I)

In another preferred option chiral amines are selected from more complicated compounds, which are polysubstituted and optionally two or all three among $R_3$, $R_4$ and $R_5$ form a ring structure like piperidine, pyrrolidine, piperazine, morpholine, quinuclidine. Such more complicated compounds are easily available if they are selected from natural compounds such as alkaloids, preferably selected without being limited to nicotine, brucine, ephedrine, cinchonidine, cinchonine, quinidine, quinine, spartein.

Given that the enantiomerically resolved compound of formula I is preferably in (*R*)-configuration, it is desirable that the used chiral amine $NR_3R_4R_5$ is present in (*R)*- and/or (*S)*-configurations and is able to provide isolable salt of compound of formula I of (*R*)-configuration.

In order to beneficially influence desired resolution of the diastereomeric salts by appropriate conditions, the step (b) of a forming diastereomeric salt in the process according to the present invention includes the sub-steps of heating a solution of the enantiomeric mixture of the compound of formula I of step (a), then adding the chiral amine at a heated state to the enantiomeric mixture under stirring, preferably vigorous stirring, to obtain a clear solution.

The diastereomeric salts obtained in the process of the present invention usually yields two salts with different solubility. The formed diastereomeric salts of the compound of formula II can then be readily separated, suitably with crystallization and notably by fractional crystallization. Fractional crystallization was preferably performed by cooling to the temperature of less than 25 °C, preferably less than 15 °C and collecting crystalline fractions. The sub-step of cooling was preferably carried out by slow cooling, e.g. not faster than about 10 °C/min, preferably at about 3-7 °C/min.

The resolution of the diastereomeric salts obtained in the process of the present invention preferably yields the desired diastereomerically resolved salt form in a diastereomeric excess (*de)* of at least 60%, preferably of at least 80%.The diastereoisomerical purity is further enhanced by preferably carrying out one or more crystallizations of the enantiomerically resolved compound of formula II. A solvent which can be used for either or both, for obtaining the solution of the enantiomeric mixture and/or for the recrystallization of enantiomerically resolved compound of formula I, is selected from the group consisting of ethyl acetate, isopropanol, isobutanol, ethanol, methanol, mixture of alcohols

and water, preferably ethyl acetate and isopropanol. The obtained diastereomeric salts were preferably optically pure.

Subsequent to optically resolving the diastereomeric salts, in step (c) the purposively selected optically resolved diastereomeric salt of formula II into the corresponding enantiomerically resolved compound of formula I is carried out by liberating the free acid form by acidification. The liberation of the thus enantiomerically enriched compound of formula I can be suitably carried out by known means such as by acidification by appropriate acids such as hydrochloric acid, acetic acid, phosphoric acid, carbonic acid, preferably hydrochloric acid.

The compound of formula I obtained in the process of the present invention can, if desired, subsequently be further modified. In a preferred option the acidification of step (c) and further modification of the compound of formula I is performed in one pot.

The process according to the present invention may allow to reuse the preliminarily wasted undesired diastereomeric salt forms (i.e. those other than the desired one optically resolved in step (b)), as these compounds, optionally after being first converted into the corresponding liberated free acid forms, are amenable to re-racemization by dehydrogenation-hydrogenation processes. As dehydrogenation-hydrogenation processes suitable for racemization, the following can be referred to for example:

Kim, Y.; Park, J.; Kim, M-J., ChemCatChem, 2011, 3, 271-277.
Parvulescu, A. N.; Jacobs, P. A.; De Vos, D. E. Adv. Synth. Catal. 2008, 113-121.
Kim, M-J.; Kim, W-H.; Han, K.; Choi, Y-K.; Park, J. Org. Lett, 2007, 91157-1159.
Pamies, O.; Backwall, J. E. Chem. Rev. 2003, 103, 3247-3262.

Possible and exemplified useful intermediate diastereomeric salt compounds of formula II include, without being limited to the following compound Ia" to Ip", wherein the stereocenter marked by * is in either (*R*)- or (*S*)-configuration, and/or wherein the chiral amine $NR_3R_4R_5$ is in either (*R*)- or (*S*)-configuration, preferably both the stereocenter marked by * and the chiral amine are in (*R*)-configuration:

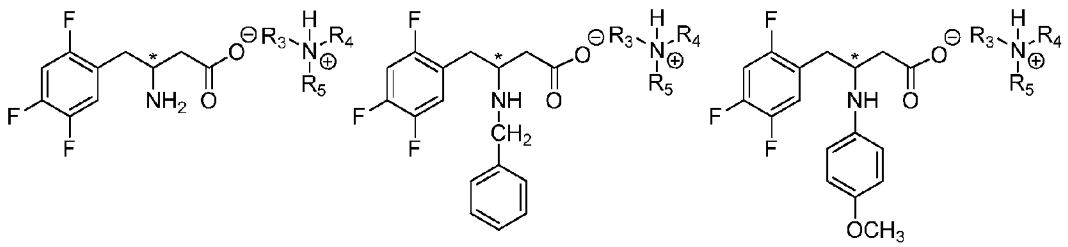

Ia" Ib" Ic"

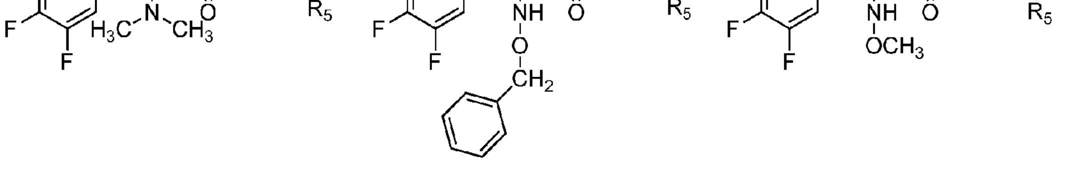

Id" Ie" If"

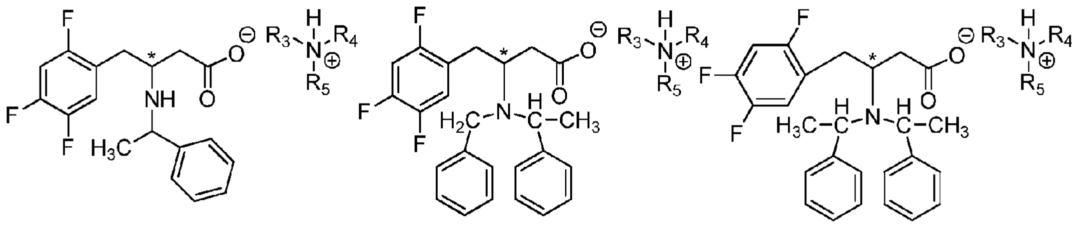

Ig''　　Ih''　　Ii''

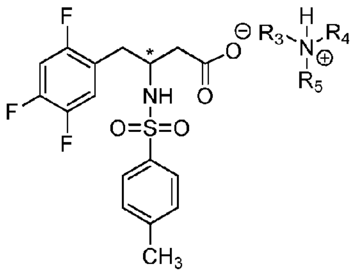

Ij''

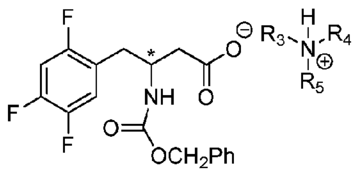

Ik''

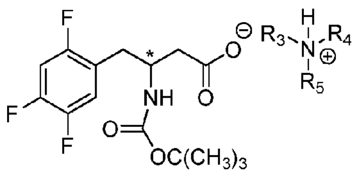

Il''

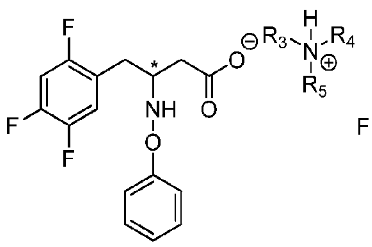

Im''

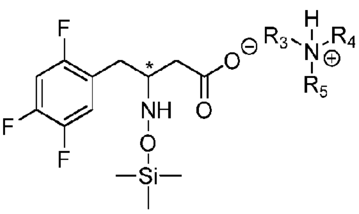

In''

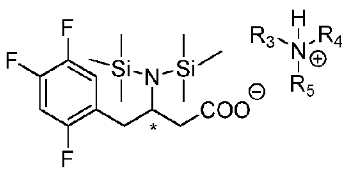

Io''

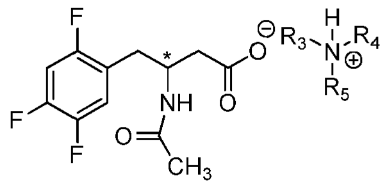

Ip''

The enantiomerically resolved compound of formula I obtained by the process according to the present invention can, if desired, subsequently be further modified or used. For example, another salt of the then enantiomerically resolved compound of formula I can be formed, preferably a pharmaceutically acceptable salt. Another preferred embodiment is that the enantiomerically resolved compound of formula I is further structurally modified to finally obtain a desired active pharmaceutically active ingredient.

For example, a possible and useful structural modification involves converting the carboxylic group in the enantiomerically resolved compound of formula I to an activated carboxylic group, in order to then couple the compound to other structural moieties such as ester groups or amide groups for obtaining a compound or intermediate and thus eventually an active pharmaceutical compound of interest belonging to the class of β-aminobutyryl compounds having γ-bound aryl groups. Suitable activations include for example, without being limited to,

(i) reaction with a sulfur, phosphorous, oxalyl or phosgene halogen derivative to obtain respective halogenide $COX_1$, wherein $X_1$ is chloro, bromo, preferably chloro; or
(ii) reaction with acyl chlorides, preferably pivaloyl chloride, or chloroformates to obtain an anhydride in which the carboxy OH-group is modified by

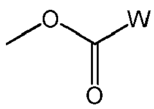

wherein W is $C_1$-$C_6$-alkyl or $C_1C_6$-alkoxy, unsubstituted or substituted benzyloxy; or
(iii) by reaction with coupling reagents which are ordinary used in peptide chemistry to obtain in situ formed intermediates, wherein the coupling reagents are selected from the group consisting of:

carbodiimides, preferably from *N*,*N*'-dicyclohexylcarbodiimide (DCCI), *N*,*N*'-diisopropylcarbodiimide (DPCI), or *N*-ethyl-*N*'-(3-dimethyaminopropyl) carbodiimide (EDC), with or without 1-hydroxybenztriazole,
activated ureas preferably *O*-(benzotriazol-1-yl)-*N*,*N*,*N*',*N*'-tetramethyluronium hexafluorophosphate (HBTU), *O*-(benzotriazol-1-yl)-*N*,*N*,*N*',*N*'-tetramethyluronium tetrafluoroborate (TBTU), and *O*-(7-aza-benzotriazol-1-yl)-*N*,*N*,*N*',*N*'-tetramethyl uronium hexafluorophosphate (HATU)
carbonyldiimidazole,
1-methylpyridinium iodide.

To be illustrated when Ar has the preferred structure of

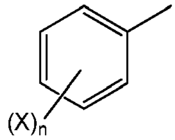

the obtained optically resolved and carboxy group activated compound would be as shown by the structures of formulae VII and VIII below, respectively:

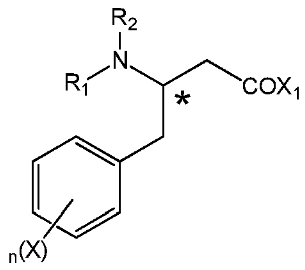

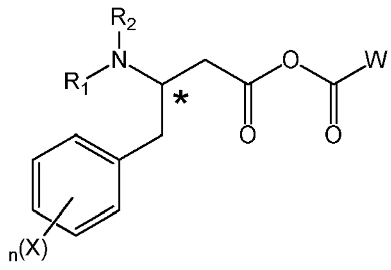

VII VIII

wherein the stereogenic center marked with an * is either in *(R)*- or *(S)*-configuration at marked center depending on the desired optical resolution, preferably is in *(R)*- configuration; and $R_1$, $R_2$, $X_1$ and W are as defined above.

According to a preferred embodiment, the activated compound obtained in either one of (i), (ii) or (iii) is reacted with a compound of formula IX

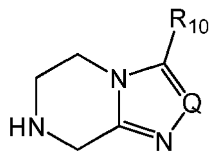

IX

wherein Q is N, CH, C-$CF_3$, or C-phenyl, preferably N, and $R_{10}$ is H, $C_1$-$C_4$-alkyl or fluorinated $C_1$-$C_2$-alkyl, preferably trifluoromethyl to give a compound of formula IXa

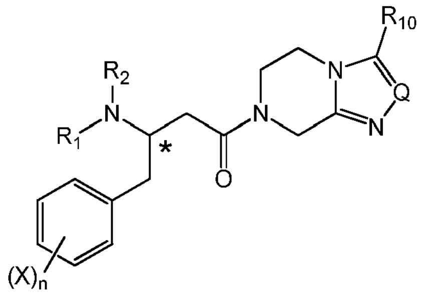

IXa

wherein the stereogenic center marked with an * is either in *(R)*- or *(S)*-configuration at marked center depending on the desired optical resolution, preferably is in *(R)*- configuration; and Ar is as defined above;
and subsequently $R_1$ and/or $R_2$ groups are removed by method known to a skilled person, such as benzyl and substituted benzyl groups can be removed by catalytic hydrogenation, acyl and sulfonyl groups by hydrolytic reactions, silyl groups by fluoride decoupling and acidic deprotection, alkyloxy by reductions characteristic for conversion of hydroxylamines to amines, finally obtaining the compound of formula X :

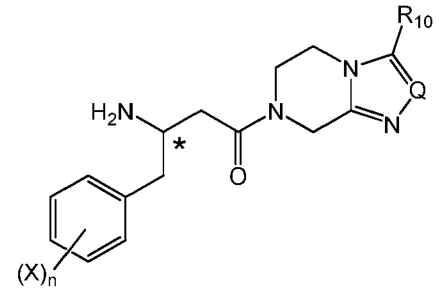

X

wherein the stereogenic center marked with an * is either in *(R)*- or *(S)*-configuration at marked center depending on the desired optical resolution, preferably is in *(R)*-configuration, and X, n, Q and $R_{10}$ are as defined above.

When all of X, n, Q and $R_{10}$ are defined by their preferred definitions as noted above, sitagliptin in a desired (*R*)-configuration can be efficiently obtained by the process according to the present invention.

Thus, the above described processes can be used in an overall process for the preparation of antidiabetic agents, in particular (*R*)-3-amino-1-[3-(trifluoromethyl)-5,6,7,8-tetrahydro[1,2,4]triazol[4,3-a]pyrazin-7-yl]-4-(2,4,5-trifluorphenyl)butan-1-one.

As a specific example illustrative to show the principle of the inventive process of optical resolution via suitable diastereomeric salts, the following scheme illustrates a preparation of representative diastereoisomeric amine salts (**2a**) and (**7a**) which are correspondingly useful to obtain e.g. optical pure 3-(benzyloxy)amino)-4-(2,4,5-trifluorophenyl)butanoic acid (**2b**) and 3-acetamido-(2,4,5-trifluorophenyl)butanoic acid (**7b**) as possible key intermediates in the sitagliptin preparation process (Scheme 2):

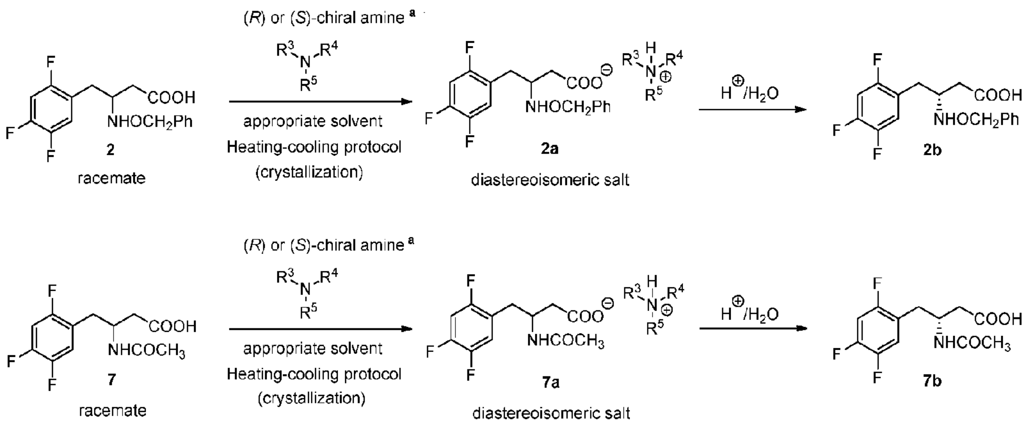


[a] (R) or (S) chiral amine, for example:
benzyl-α-methyl-benzyl amine; methyl-benzyl-amine; nicotine; brucine; ephedrin; 1-phenyl-propyl amine; 2-phenylglicinol

**Scheme 2**

Further illustratively, the following scheme shows as how the principle of the inventive process of optical resolution via suitable diastereomeric salts is suitable included in a whole synthesis scheme for obtaining optically pure β-amino acid type active pharmaceutical ingredients and intermediates thereof, here again with the example of optical pure sitagliptin (Scheme 3):

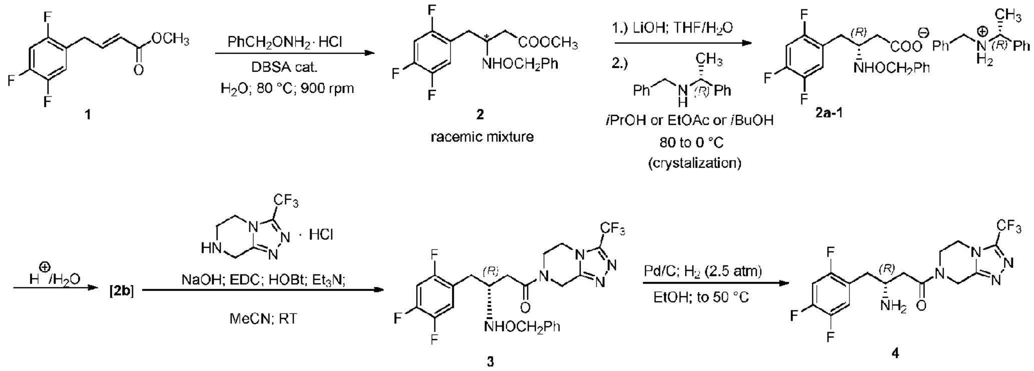


**Scheme 3**

As a further exemplified, but specifically illustrative embodiment, the following scheme shows another way how the principle of the inventive process of optical resolution via suitable diastereomeric salts can be suitable included in a whole synthesis scheme, here again for obtaining optically pure sitagliptin (Scheme 4):

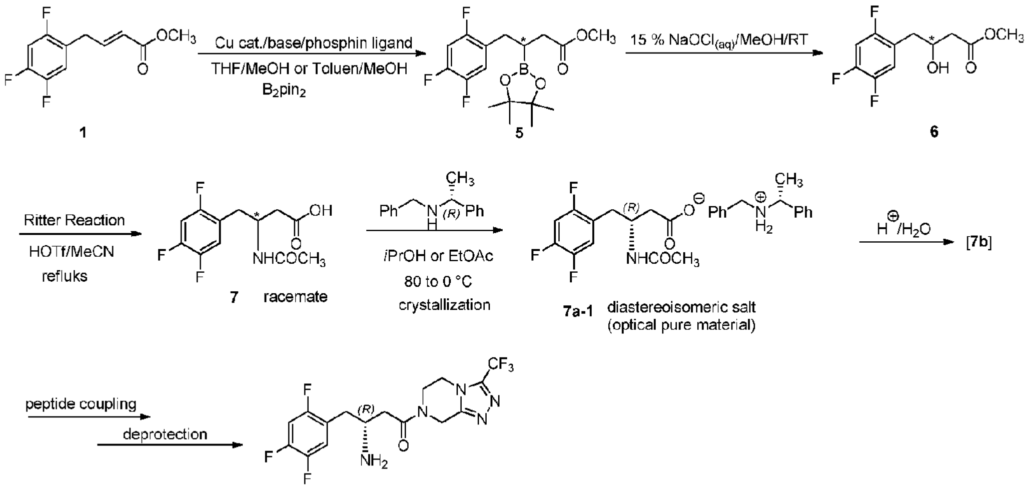


**Scheme 4**

## Examples

The present invention will now be described in further detail by way of purely illustrative examples, without limiting the invention thereto.

Exemplified Synthesis of starting compounds or intermediates suitably obtained before the optical resolution approaches are applied: Starting compound (**1**) was prepared according to procedure published from Liu, F.; et. al. J. Chem. Res. 2010, 34, 230-232.

Synthesis Example 1. Preparation of methyl 3-(benzyloxyamino)-4-(2,4,5-trifluorophenyl) butanoate (**2**) from (**1**) in the presence of surfactant-type Brønsted acid dodecybenzenesulfonic acid (DBSA) in water as reaction medium:

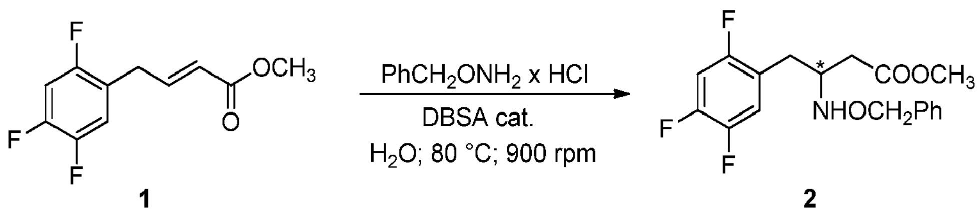


1 2

Into a flask equipped with a magnetic stir bar were placed *O*-benzylhydroxylamine hydrochloride (37.8 mmol, 6.10 g), NaOH (1.50 g) and deionized water was added. After 15 min DBSA (5.4 mmol, 1.80 g) was added and reaction system was heated to 60 °C. Afterwards methyl (*E*)-4-(2,4,5-trifluorophenyl)-but-2-enoate (**1**) (27 mmol, 6.20 g) was slowly dropped and such reaction mixture was intensively stirred (900 rpm) at 80 °C for 30 hours. Reaction mixture was diluted with saturated aqueous solution of $NaHCO_3$ and extracted with EtOAc. The combined organic phases were finally washed with brine, dried over anhydrous $Na_2SO_4$ and organic solvent was evaporated under reduced pressure. The obtained crude product (**2**) was purified with column chromatography ($SiO_2$ *n*-hexane : ethyl acetate = 2 : 1 gradient elution) or distillation under reduced pressure to obtain (11.2 g, 94% yield) of pure product (**2**). The product was finally analyzed and confirmed with $^{1}H$, $^{19}F$ and $^{13}C$ NMR analysis. $^{1}H$ NMR (500 MHz, $CDCl_3$, ppm) $\delta$ 7.35-7.45 (m, 5ArH), 7.10 (m, 1ArH), 6.90 (m, 1ArH), 5.85 (bs, NH), 4.65 (s, 2H), 3.65 (s, 3H), 3.45-3.55 (m, 1 H), 2.90 (ddd, $J$ = 14 Hz , $J$ = 8.7 Hz, $J$ = 1.4 Hz, 1 H), 2.75 (ddd, $J$ = 14 Hz, $J$ = 7.8 Hz, $J$ = 1.4 Hz, 1H), 2.50 (dd, $J$ = 16 Hz, $J$ = 7.8 Hz, 1H), 2.45 (dd, $J$ = 16 Hz, $J$ = 5.2 Hz, 1 H). $^{13}C$ (125 MHz, $CDCl_3$, ppm) $\delta$ 30.6, 36.0, 51.6, 57.5, 75.2, 105.4 (m), 119.1 (m), 121.5 (m), 127.3, 128.5, 137.5, 145.7 (m, C-F), 149.7 (m, C-F), 157.0 (m, C-F), 172.2 (CO). $^{19}F$ NMR (470 MHz, $CDCl_3$, ppm) $\delta$ -119.9 (m), -136.9 (m), -143.9 (m).

Synthesis Example 2: Synthesis of methyl 3-(benzyloxyamino)-4-(2,4,5-trifluorophenyl)butanoate (**2**) from (**1**) in water in the presence of 2,2,2-trifluoroethanol (TFE) as promoter:

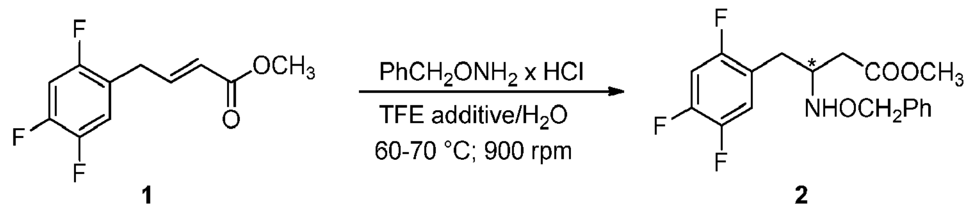


1 2

Into a flask equipped with an magnetic stir bar were placed *O*-benzylhydroxylamine hydrochloride (0.7 mmol, 112 mg), NaOH (0.7 mmol, 28 mg) and deionized water was added. After 15 min TFE (2.5 mmol, 180 mL) was added and such reaction system was heated to 60 °C. Afterwards methyl (*E*)-4-(2,4,5-trifluorophenyl)-but-2-enoate (**1**) (0.5 mmol, 115 mg) was slowly added and final reaction mixture was intensively stirred (900 rpm) at 60 °C for 24 hours. Reaction mixture was diluted with saturated aqueous solution of NaCl (3.5 mL) and extracted with EtOAc (2 $\times$ 25 mL). The combined organic phases were dried over anhydrous $Na_2SO_4$ and organic solvent was evaporated under reduced pressure. The obtained product (**2**) (75 mg, 42% yield) was analyzed and confirmed with $^{1}H$, $^{19}F$ and $^{13}C$ NMR analysis.

Synthesis Example 3: Synthesis of methyl 3-(benzyloxy-amino)-4-(2,4,5-trifluorophenyl)butanoate (**2**) from (**1**) in water in the presence of SDS and TFE as promoters:

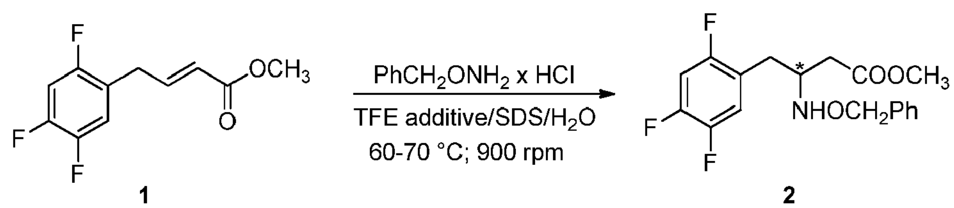


1 2

Into a flask equipped with a magnetic stir bar were placed *O*-benzylhydroxylamine hydrochloride (0.7 mmol, 112 mg), NaOH (0.7 mmol, 28 mg) and deonized water (2.5 mL). Afterward catalytic amount of SDS (0.04 mmol, 12

mg) was added and such reaction system was stirred at ambient temperature for 10 minutes. In the next step methyl (*E*)-4-(2,4,S-trifluorophenyl)-but-2-enoate (**1**) was slowly dropped into the reaction mixture and system was heated to 60 °C. Finally the TFE (5 equiv.; 180 µL) was added and such reaction mixture was vigorously stirred (800 rpm) at 60 °C for 15 hours. Reaction mixture was diluted with saturated aqueous solution of NaCl (3.5 mL) and gently extracted with EtOAc (2 × 30 mL). The combined organic phases were dried over anhydrous $Na_2SO_4$ and organic solvent was evaporated under reduced pressure. The obtained product (**2**) (123 mg, 70.5% yield) was analyzed and confirmed with $^1H$, $^{19}F$ and $^{13}C$ NMR analysis.

Synthesis Example 4: Synthesis of methyl 3-(benzyloxyamino)-4-(2,4,5-trifluorophenyl)butanoate (**2**) from (**1**) in water in the presence of $Sc(OTf)_3$ and SDS as catalysts:

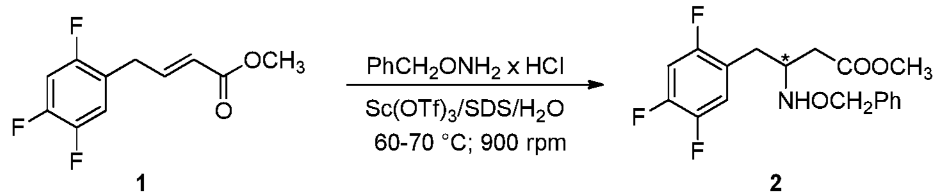


**1** **2**

Into a flask equipped with a magnetic stir bar were placed *O*-benzylhydroxylamine hydrochloride (0.7 mmol, 112 mg), NaOH (0.7 mmol, 28 mg) and deonized water (2.5 mL). Afterward SDS (0.1 mmol; 29 mg; 20 mol% according to substrate) and $Sc(OTf)_3$ (0.1 mmol; 49 mg; 20 mol%,) and such reaction system was heated to 60 °C. Substrate methyl (*E*)-4-(2,4,S-trifluorophenyl)-but-2-enoate (**2**) (0.5 mmol, 115 mg) was slowly added and final reaction mixture was intensively stirred (900 rpm) at 60 °C for 24 hours. Reaction mixture was diluted with saturated aqueous solution of NaCl (3.5 mL) and extracted with EtOAc (2 × 25 mL). The combined organic phases were dried over anhydrous $Na_2SO_4$ and organic solvent was evaporated under reduced pressure. The obtained product (**1**) (110 mg, 62% yield) was analyzed and confirmed with $^1H$, $^{19}F$ and $^{13}C$ NMR analysis.

Synthesis Example 5: Copper-catalyzed synthesis of methyl-3-(4,4,5,5-tetramethyl-1,3,2-dioxaborolan-2-yl)-4-(2,4,5-trifluorophenyl)butanoate (**5**) from (**1**) in the presence of monophosphine ligands:

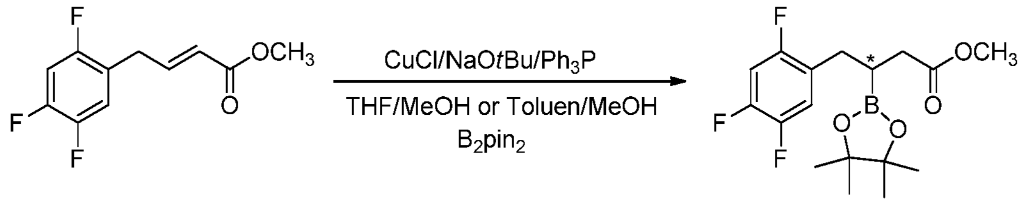


**1** **5**

In a two-necked round bottom flask were placed CuCl (0.10 mmol, 10.2 mg), NaO*t*Bu (0.15 mmol, 14.42 mg) and $Ph_3P$ (0.1 mmol, 26.23 mg) under the nitrogen. Afterward 1.5 mL of anhydrous THF was added and the reaction mixture was stirred (600 rpm) at ambient temperature for 45 min. Than the solution of bis(pinacolato)diboron (1.1 mmol, 280.0 mg, 1.10 equiv.) in 0.5 mL of THF was slowly dropped into the reaction mixture and after 30 min α,β unsaturated ester (*E*)-methyl-4-(2,4,5-trifluorophenyl)-but-2-enoate (**1**) (1 mmol, 230.6 mg) dissolved in 0.5 mL THF was added. The reaction mixture was stirred at ambient temperature for 30 min and afterward MeOH (1.25 mL; THF : MeOH = 2 : 1) or $H_2O$ (1.25 mL; THF : $H_2O$ = 2 : 1) were added. The reaction system was maintained at ambient temperature and stirred for 24 hours. The reaction mixture was concentrated under the reduced pressure, than brine (10 mL) was added and mixture was transferred to a separating funnel. The aqueous layer was extracted with two portion of EtOAc (2 × 30 mL). Combined organic layers were washed with brine (30 mL), dried over $Na_2SO_4$ and organic solvent was removed under the reduced pressure. The obtained crude brown product was purified with flash chromatography ($SiO_2$; EtOAc) to obtain 310 mg of yellow liquid (86% yield) (**5**) as determined with $^1H$, $^{11}B$, $^{13}C$ NMR and MS analysis.

$^1H$ NMR (500 MHz, $CDCl_3$, ppm) δ 7.01-7.05 (m, 1 H), 6.80-6.86 (m, 1 H), 3.65 (s, 3H), 2.75 (dd, *J* = 15 Hz, *J* = 5 Hz, 1 H), 2.61 (dd, *J* = 15 Hz, *J* = 5 Hz, 1 H), 2.37 (d, *J* = 10 Hz, 2H), 1.60 (pentet, 1 H), 1.20 (m, 12H).

$^{13}C$ NMR (125 MHz, $CDCl_3$, ppm) δ 24.5, 28.4, 34.4, 53.3, 83.4, 105.0 (dd, *J* = 28.8 Hz, *J* = 21.3 Hz), 118.5 (dd, *J* = 20.2 Hz, *J* = 6.3 Hz), 124.5 (m), 147.3 (m), 149.4 (m), 156.0 (dd, *J* = 242.5 Hz, *J* = 2.5 Hz), 173.7.

$^{11}B$ (160 MHz, $CDCl_3$, ppm) δ 33.6 (bs).

Synthesis Example 6: Copper-catalyzed synthesis of methyl-3-(4,4,5,5-tetramethyl-1,3,2-dioxaborolan-2-yl)-4-(2,4,5-trifluorophenyl)butanoate (**5**) from (**1**) in the presence of diphosphine ligands:

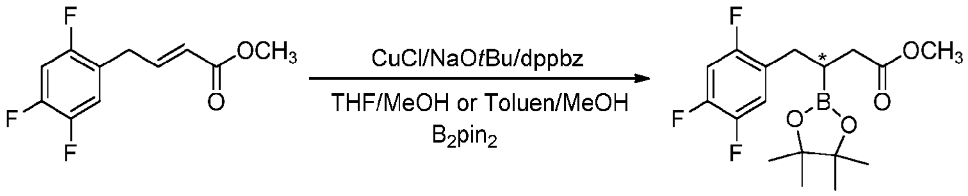


1 5

In a two-necked round bottom flask were placed CuCl (0.1 mmol, 10.2 mg), NaO*t*Bu (0.15 mmol, 14.42 mg) and 1,2-diphenylphosphinobenzene (dppbz; 0.1 mmol, 44.6 mg) or 1,1-bis(di-*tert* butylphosphino)ferrocene (dtpf; 0.1 mmol, 47.4 mg) under the nitrogen. Afterward 1.5 mL of anhydrous THF was added and the reaction mixture was stirred (600 rpm) at ambient temperature for 45 min. Than the solution of bis(pinacolato)diboron (1.1 mmol, 280 mg, 1.10 equiv.) in 0.5 mL of THF was slowly dropped into the reaction mixture and after 30 min $\alpha,\beta$ unsaturated ester (*E*)-methyl-4-(2,4,5-trifluorophenyl)-but-2-enoate (**1**) (1.00 mmol, 230.6 mg) dissolved in 0.5 mL THF was added. The reaction mixture was stirred at ambient temperature for 30 min and afterward MeOH (1.25 mL; THF : MeOH = 2 : 1) were added. The reaction system was maintained at ambient temperature and stirred for 24 hours. The reaction mixture was concentrated under the reduced pressure and then brine (10 mL) was added and mixture was transferred to a separating funnel. The aqueous layer was extracted with $CH_2Cl_2$ (2 $\times$ 30 mL). Combined organic layers were washed with brine (30 mL), dried over $Na_2SO_4$ and organic solvent was removed under the reduced pressure. The obtained crude product was purified with flash chromatography ($SiO_2$; $CH_2Cl_2$) to obtain 295 mg (82% yield) of yellow liquid (**5**) as determined with $^1H$, $^{11}B$, $^{13}C$ NMR and MS analysis. The conversion of starting material (**1**) to (**5**) was more than 95% as determined with $^1H$ NMR analysis. The $\beta$-boration of (**1**) catalyzed with dtpf (8-10 mol%) was successfully tested also on the larger scale experiments (15-100 mmol scale).

Synthesis Example 7: One-pot $\beta$-boration/oxidation synthetic protocol to secondary alcohol methyl-3-hydroxy-4-(2,4,5-trifluorophenyl)butanoate (**6**) from intermediate (**5**):

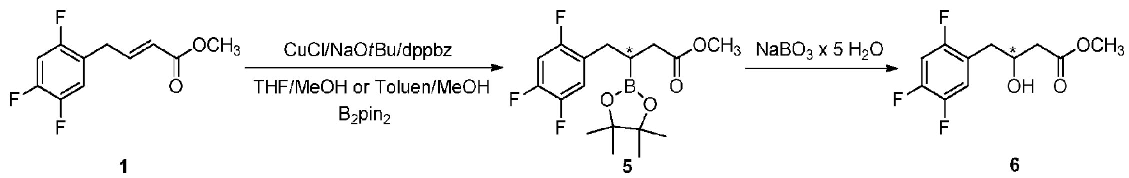


1 5 6

After the completion of the $\beta$-boration of the starting compound (**1**) the reaction mixture was diluted with THF (5 mL) and $H_2O$ (7.5 mL). Afterward sodium perborate hydrate (**4** mmol, 399.2 mg, 4.0 equiv.) were sequentially added into the reaction mixture and the reaction system was vigorously stirred at ambient temperature for 24 hours under air. The reaction mixture was concentrated under the reduced pressure, than extracted with EtOAc (3 $\times$ 20 mL) and combined organic layers were gently washed with brine (30 mL) and dried over anhydrous $Na_2SO_4$. After the removal of the organic solvent under reduced pressure, a crude dark yellow liquid was obtained which was further purified with flash chromatography ($SiO_2$; EtOAc) to obtain 220 mg (88% yield) of a crude yellow viscous liquid (**6**). The structure of (**6**) was confirmed by $^1H$ and $^{13}C$ NMR analysis.

$^1H$ NMR (500 MHz, $CDCl_3$, ppm) $\delta$ 7.13 (m, 1H), 6.90 (m, 1H), 4.24 (m, 1H), 3.71 (s, 3H), 3.14 (bs, OH), 2.80 (d, *J* = 5 Hz, 2H), 2.43-2.56 (m, 2H).

$^{13}C$ NMR (125 MHz, $CDCl_3$, ppm) $\delta$ 34.9, 40.2, 51.9, 67.5, 105.3 (dd, *J* = 28.8 Hz, *J* = 21.3 Hz), 119.4 (dd, *J* = 18.8 Hz, *J* = 6.3 Hz), 121.1 (d, *J* = 18.8 Hz), 145.6 (m), 147.6 (m), 156.0 (dd, *J* = 243 Hz, *J* = 7.0 Hz), 173.

Synthesis Example 8: The synthesis of methyl-3-hydroxy-4-(2,4,5-trifluorophenyl)butanoate (**6**) from (**5**) using aqueous solution of sodium hypochlorite (NaOCl) as oxidizing agent:

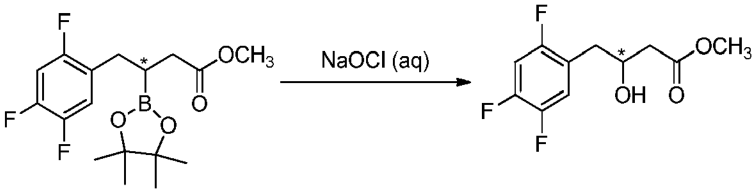


5 6

The organoborane intermediate methyl-3-(4,4,5,5-tetramethyl-1,3,2-dioxaborolan-2-yl)-4-(2,4,5-trifluorophenyl)butanoate (**5**) (1 mmol, 358 mg) was dissolved in 2 mL of $CH_3OH$ and stirred at ambient temperature under air atmosphere for a few minutes. Afterward the bleach (12-15% aqueous solution of NaOCl, 2.5 equiv. according to 5) was slowly dropping into the reaction system and vigorously stirred for 8 hours. The reaction mixture was first concentrated under the reduced pressure and organic residue was extracted with EtOAc (2 × 15 mL). The combined organic layers were than washed with saturated aqueous solution of $NaHCO_3$ dried over anhydrous $Mg_2SO_4$ and organic solvent was evaporated. The obtained crude product was purified with flash chromatography ($SiO_2$; EtOAc) to obtain 225 mg (90.7% yield) of (**6**) as determined with $^1$H NMR spectroscopy.

Synthesis Example 9: Synthesis of methyl 3-acetamido-4-(2,4,5-trifluorophenyl)butanoate (7):

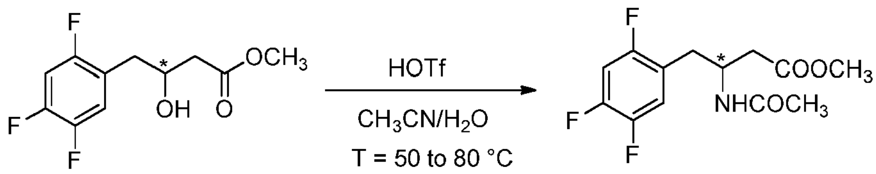


6 7

In a thick-walled glass vial equipped with a magnetic stir bar was placed methyl 3-hydroxy-4-(2,4,5-trifluorophenyl)butanoate (**6**) (2 mmol 115 mg) and than acetonitrile (3 mL) was added to obtain clear solution. Afterwards trifluoromethanesulfonic acid (15 mmol, 1.32 mL) was slowly added through the septum followed by addition of water (15 mmol, 0.3 mL) and reaction mixture was slowly heated to 80 °C. Such reaction system was stirred for 16 hours at 80 °C. Solvent was first evaporated under reduced pressure, organic residue was neutralized with saturated aqueous solution of $NaHCO_3$ (4 mL) and gently extracted with EtOAc (2 × 30 mL). The combined organic phases were dried over $MgSO_4$ and the solvent was evaporated under reduced pressure. The crude product was purified with column chromatography ($SiO_2$ *n*-hexane : ethyl acetate = 1 : 1) to obtain oily product (368 mg; 63.6 % yield) which was analyzed and determined with $^1$H, $^{13}$C NMR and MS analysis.

$^1$H NMR (500 MHz, $CDCl_3$, ppm) δ 7.05 (m, 1H), 6.90 (m, 1H), 5.41 (pentet, 1H), 3.70 (s, 3H), 3.01 (dd, *J* = 15 Hz, *J* = 5 Hz, 1 H), 2.90 (dd, *J* = 15 Hz, *J* = 5 Hz, 1 H), 2.60 (m, 2H), 2.01 (s, 3H).

$^{13}$C NMR (125 MHz, $CDCl_3$, ppm) δ 20.9, 32.4, 38.0, 51.9, 69.5, 105.5 (dd, *J* = 28.8 Hz, *J* = 21.3 Hz), 119.3 (dd, *J* = 18.8 Hz, *J* = 6 Hz), 120.3 (d, *J* = 18.8 Hz), 146.3 (m), 148.1 (m), 156.0 (m), 169.9 (CO), 170.3 (CO).

MS (Cl) m/z (%) 291 (M + 1, 25%), 259 (70%), 231 (100%), 199 (76%).

Synthesis Example 10: Preparation of (*R*)-3-((benzyloxy)amino)-4-(2,4,5-trifluorophenyl)butanoic acid (*R*)-(+)-*N*-benzyl-α-methylbenzylamine salt (**2a-1**) from ethyl acetate solution:

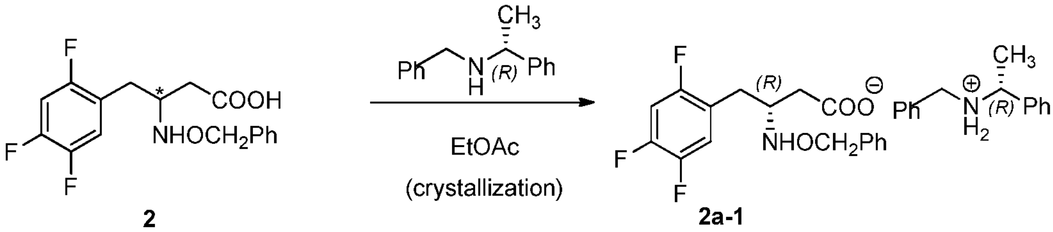


Racemic mixture of 3-((benzyloxy)amino)-4-(2,4,5-trifluorophenyl)butanoic acid **2** (0.6 g, 1.8 mmol) was dissolved in EtOAc (2 mL) and slowly heated to 80 °C. Within 15 min a solution of (*R*)-(+)-*N*-benzyl-α-methylbenzylamine (1.8 mmol; 398.4 mg in 1 mL EtOAc) was added and such reaction mixture was vigorously stirred for 45 min. The clear 0.5 M final solution was then slowly (5 °C/min) cooled first to 25 °C and then slowly to 12 °C and overnight the product crystallized as voluminous white solid which was filtered off. The remained filtrate solution was again heated to 30 °C and quickly cooled to 0 to 3 °C. The white precipitates (crystalline fractions) was collected by filtration, crystalline fractions were analyzed using HPLC analysis and recrystallized from EtOAc or *i*PrOH to obtain (396 mg; 37%) of final material with high optical purity (> 98%).

$^1$H NMR (500 MHz, DMSO, ppm) δ 7.10-7.50 (m, 17 ArH), 4.60 (bs, 2H), 4.30 (bs, NH), 3.80 (q, 1 H), 3.50 (d, 2H), 3.35 (m, 1 H), 2.80-2.90 (dd, 1 H), 2.60-2.70 (dd, 1 H), 2.30-2.40 (dd, 1 H), 2.15-2.25 (dd, 1 H), 1.25 (d, 3H).

IR (KBr): v= 3350-3550 (broad, medium), 1560 (strong), 1516 (strong), 1397 (medium), 1150 (medium) $cm^{-1}$.

Synthesis Example 11. Preparation of (*R*)-3-((benzyloxy)amino)-4-(2,4,5-trifluorophenyl)butanoic acid (*R*)-(+)-*N*-benzyl-α-methylbenzylamine salt (**2a-1**) from isopropyl alcohol solution:

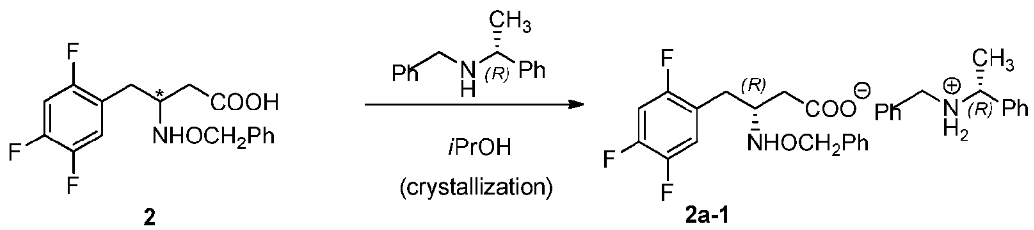


Racemic mixture of 3-((benzyloxy)amino)-4-(2,4,5-trifluorophenyl)butanoic acid **2** (0.5 g, 1.5 mmol) was dissolved in isopropyl alcohol (8 mL) and slowly heated to 80 °C. Within 15 min a solution of (*R*)-(+)-*N*-benzyl-α-methylbenzylamine (1.5 mmol; 332 mg in 2 mL *i*PrOH×) was added and such reaction mixture was vigorously stirred for 45 min. The clear solution was then slowly (5 °C/min) cooled first to 25 °C and then slowly to 7 °C and overnight the product crystallized as voluminous white solid which was filtered off. The remained filtrate solution was again heated to 30 °C and quickly cooled to 0 to 3 °C. The white precipitates (crystalline fractions) was collected by filtration, crystalline fractions were analyzed using HPLC analysis and recrystallized from EtOAc or *i*PrOH to obtain (360 mg; 34%) of final material with high optical purity (> 98%).

Synthesis Example 12. Transformation of (*R*)-3-((benzyloxy)amino)-4-(2,4,5-trifluorophenyl)butanoic acid (*R*)-(+)-α-methylbenzylamine salt (**2a-2**) to optical pure (*R*)-3-((benzyloxy)amino)-4-(2,4,5-trifluorophenyl)butanoic acid (**2b**):

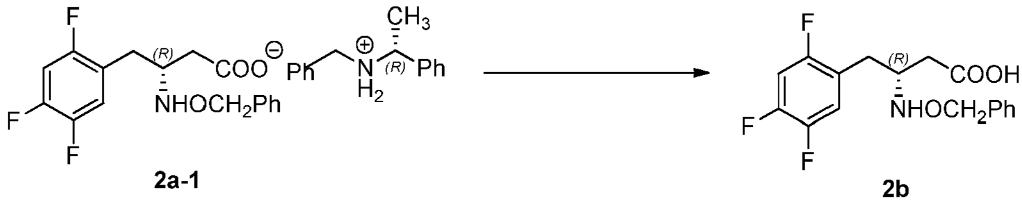

2a-1 2b

The 0.5 g of diastereoisomeric salt **2a-1** was suspended under vigorous stirring in diethyl ether (7 mL). Afterwards 2 equivalents (0.90 mL) of 2 M aqueous solution of hydrochloric acid was slowly dropped into reaction mixture and stirred for an hour. The organic layer was separated and washed with small amount of saturated aqueous solution of $NaHCO_3$ Aqueous phase was one more time extracted with diethyl ether and all combined organic phases was dried over anhydrous $Na_2SO_4$. After evaporation of solvent optical pure free carboxylic acid **2b** was obtained and determined with HPLC analysis.

Synthesis Example 13. Preparation of (*R*)-3-((benzyloxy)amino)-4-(2,4,5-trifluorophenyl)butanoic acid (*R*)-(+)-α-methylbenzylamine salt (**2a-2**) from isopropy alcohol solution:

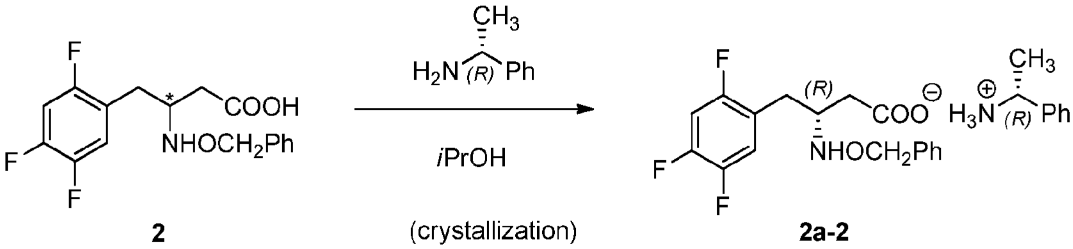


(crystallization)

**2** **2a-2**

Racemic mixture of 3-((benzyloxy)amino)-4-(2,4,5-trifluorophenyl)butanoic acid **2** (0.6 g, 1.8 mmol) was dissolved in *i*PrOH (2 mL) and slowly heated to 80 °C. Within 15 min a solution of (*R*)-(+)-α-methylbenzylamine (1.8 mmol; 218 mg in 1 mL *i*PrOH) was added and such reaction mixture was vigorously stirred for 45 min. The clear solution was then slowly (5 °C/min) cooled first to 25 °C and then slowly to 3 °C and overnight the product crystallized as voluminous white solid which was filtered off. The solution was again heated to 30 °C and quickly cooled to 0 °C. The white precipitates (crystalline fractions) was collected by filtration, crystalline fractions were analyzed using HPLC analysis and recrystallized from EtOAc or *i*PrOH to obtain (340 mg; 37%) of final material with high optical purity (> 98%).

IR (KBr): v= 3400-3550 (broad, medium), 1560 (medium), 1519 (strong), 1399 (medium), 1207 (medium), 1150 (medium) $cm^{-1}$.

## Claims

**1.** A process for preparing enantiomerically resolved compound of formula I

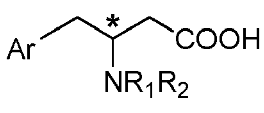

**I**

wherein the stereogenic center marked by * is in *(R)* or in *(S)* configuration in enantiomerically resolved state; wherein $R_1$ and $R_2$ are identical or different, and are independently selected from

(i) hydrogen;

(ii) alkyl residues optionally chiral, having from 1 to 12 carbon atoms, wherein the alkyl residues are optionally aryl and/or aryloxy substituted;
(iii) alkyloxy residues optionally chiral, having from 1 to 12 carbon atoms, wherein the alkyloxy residues are optionally aryl substituted;
(iv) aryl residues optionally chiral, having from 6 to 24 carbon atoms, wherein the aryl residues are optionally alkyl and/or alkyloxy substituted;
(v) aryloxy residues optionally chiral, having from 6 to 24 carbon atoms, wherein the aryloxy residues are optionally alkyl substituted;
(vi) benzyl;
(vii) alkaloyl residues optionally chiral, having from 2 to 13 carbon atoms, wherein the alkaloyl residues are optionally aryl substituted;
(viii) aroyl residues optionally chiral, having from 7 to 25 carbon atoms, wherein the aryloxy residues are optionally alkyl substituted; and
(ix) alkoxycarbonyl residues optionally chiral, having from 2 to 13 carbon atoms;
(x) aryloxycarbonyl residues optionally chiral, having from 7 to 25 carbon atoms;
(xi) tosyl;
(xii) silyl residues optionally chiral, having from 3 to 15 carbon atoms; and
(xiii) silyloxy residues optionally chiral, having from 3 to 15 carbon atoms; and

Ar denotes respectively unsubstituted or substituted phenyl, alkyl-aryl, alkoxy-aryl,
the process comprising the steps of:

(a) providing an enantiomeric mixture of the compound of formula I

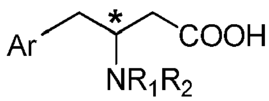

I

wherein $R_1$, $R_2$ and Ar are as defined above, and wherein * indicates a stereogenic center, present in an enantiomeric mixture;
(b) forming a diastereomeric salt with a chiral amine $NR_3R_4R_5$ to obtain a compound of formula II, wherein $R_1$, $R_2$ and Ar are as defined above and $R_3$, $R_4$ and $R_5$ are independently selected from residues to form a chiral amine, wherein two or all three among $R_3$, $R_4$ and $R_5$ may together form a ring structure, provided that at least one of $R_3$, $R_4$ and $R_5$ or said ring structure contains a chiral center;

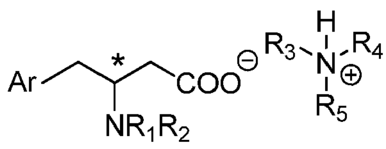

**II**

providing optical resolution of the diastereometric salt of formula II into a desired diastereomerically resolved salt form; and
(c) converting the optically resolved diastereomeric salt of formula II into enantiomerically resolved compound of formula I.

**2.** The process according to claim 1, wherein the enantiomeric mixture provided in step (a) is a racemate of both (*R*)-

and (*S)*-configurations of the compound of formula I.

**3.** The process according to claim 1 or 2, wherein the enantiomerically resolved compound of formula I has an enantiomeric excess (*ee*) of at least 60%, preferably 80%, more preferably is enantiomerically pure.

**4.** The process according to anyone of the preceding claims, wherein the enantiomeric mixture of the compound of formula I

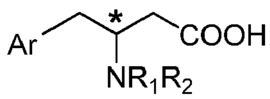

I

provided in step (a) has been previously prepared by a process comprising the steps of:

(0-1) providing a compound of formula IV,

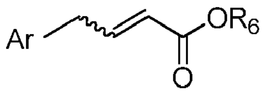

IV

wherein $R_6$ is selected from alkyl residues having from 1 to 6 carbon atoms;
(0-2) reacting the compound of formula IV with an amine of formula $HNR_1R_2$, wherein $R_1$ and $R_2$ are as defined above, in a protic solvent, particularly in water; or in a mixture of protic solvents, wherein the mixture particularly comprises water; or without adding of solvents in step (0-2);
(0-3) cleaving the ester group to obtain the compound of formula I.

**5.** The process according to anyone of the claims 1 to 3, wherein the enantiomeric mixture of the compound of formula I

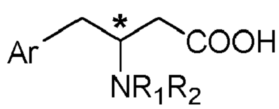

I

provided in step (a) has been previously prepared by a process comprising the steps of:

(0-1') providing a compound of formula IV

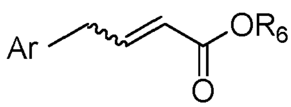

IV

wherein $R_6$ is selected from alkyl residues having from 1 to 6 carbon atoms;

(0-2') reacting the compound of formula IV with a borating agent in a suitable solvent to obtain an intermediate of formula V,

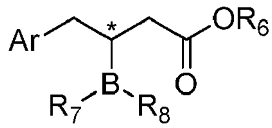

V

wherein * denotes a stereogenic center, and $R_6$ is selected from alkyl residues having from 1 to 6 carbon atoms; wherein $R_7$ and $R_8$ are identical or different, and are selected from

(i) alkyl or alkoxy residues, each having from 1 to 12 carbon atoms, wherein each alkyl or alkoxy residue is optionally aryl substituted,
(ii) aryl or aryloxy residues, each having from 6 to 14 carbon atoms, wherein each aryl or aryloxy residue is optionally alkyl substituted;
(iii) halides; and
(iv) wherein $R_7$ and $R_8$ optionally form a chiral or non-chiral 5 to 10, particularly 5 to 6, membered mono or bicyclic ring, wherein the ring is optionally substituted at least one position with an alkyl residue having from 1 to 12 carbon atoms and/or an aryl residue having from 6 to 14 carbon atoms, and wherein $R_7$ and $R_8$ optionally form an *O*-benzenedioxy residue; and

(0-3') converting the compound of formula V to the compound of formula I.

**6.** The process according to anyone of the preceding claims, wherein the structural moiety of $NR_1R_2$ is selected from the group consisting of

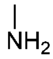

Ia

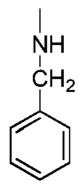

Ib

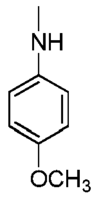

Ic

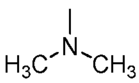

Id

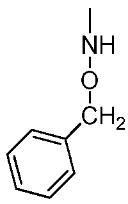

Ie

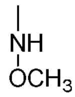

If

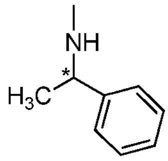

Ig

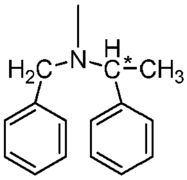

Ih

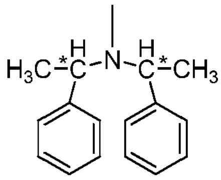

Ii

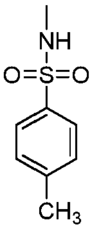

Ij

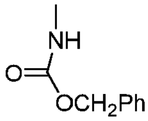

Ik

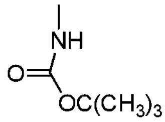

Il

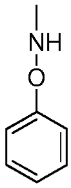

Im

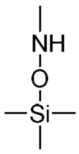

In

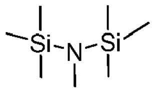

Io

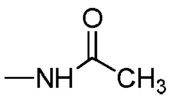

Ip

**7.** The process according to anyone of the preceding claims, wherein Ar is phenyl or substituted phenyl, preferably Ar is substituted phenyl defined of the formula

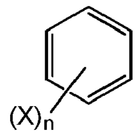

wherein X is halogen selected from fluoro, chloro, or bromo, preferably fluoro, same or different, and n is 1-4, in particular Ar is substituted phenyl defined by the formula

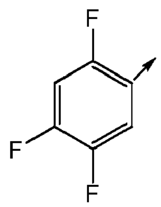

wherein the arrow indicates the binding of the Ar group to the gama($\gamma$)-carbon atom of the $\beta$-amino acid structural moiety in the compounds of formula I and II.

**8.** The process according to anyone of the preceding claims, wherein the chiral amine $NR_3R_4R_5$ , is independently selected from the group consisting of

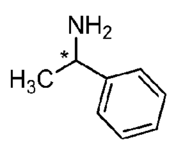

(A)

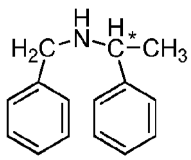

(B)

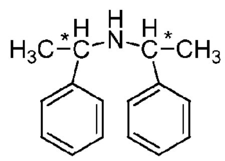

(C)

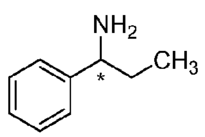

(D)

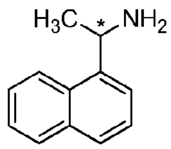

(E)

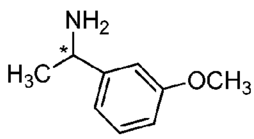

(F)

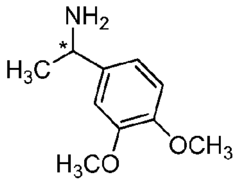
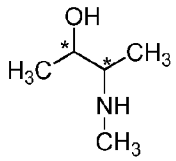
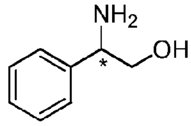

(G) (H) (I)

respectively present in (*R*)- and/or (*S*)-configurations, and nicotine, brucine, ephedrine, cinchonidine, cinchonine, quinidine, quinine, spartein.

**9.** The process according to anyone of the preceding claims, wherein step (b) of forming a diastereomeric salt includes the sub-steps of heating a solution of the enantiomeric mixture of the compound of formula I of step (a), then adding the chiral amine at a heated state to the enantiomeric mixture under stirring to obtain a clear solution, followed by controllable cooling.

**10.** The process according to claim 9, wherein the sub-step of cooling is carried out by slow cooling not faster than about 10 °C/min, preferably at about 3-7 °C/min.

**11.** The process according to anyone of the preceding claims, wherein in the step (b) enantiomerically resolved compound of formula II is recrystallized.

**12.** The process according to claim 9 or 11, wherein a solvent used for the solution of the enantiomeric mixture and/or for recrystallization of enantiomerically resolved compound of formula II is selected from the group consisting of ethyl acetate, isopropanol, isobutanol, ethanol, methanol, mixture of alcohols and water, preferably ethyl acetate and isopropanol.

**13.** The process according to anyone of the preceding claims, wherein the step (c) of converting the optically resolved diastereomeric salt of formula II into the corresponding enantiomerically resolved compound of formula I is carried out by liberating the free acid form by acidification.

**14.** The process according to anyone of the preceding claims, wherein the enantiomerically resolved compound of formula I is further structurally modified to finally obtain a desired active pharmaceutical ingredient (API), preferably to finally obtain sitagliptin.

**15.** A compound of formula II

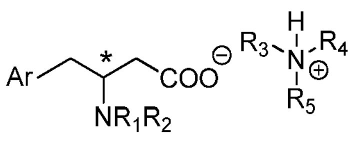

**II**

wherein * indicates a stereogenic center,
$R^1$ and $R^2$ are identical or different, and are independently selected from

(i) hydrogen;
(ii) alkyl residues optionally chiral, having from 1 to 12 carbon atoms, wherein the alkyl residues are optionally aryl and/or aryloxy substituted;
(iii) alkyloxy residues optionally chiral, having from 1 to 12 carbon atoms, wherein the alkyloxy residues are optionally aryl substituted;

(iv) aryl residues optionally chiral, having from 6 to 24 carbon atoms, wherein the aryl residues are optionally alkyl and/or alkyloxy substituted;
(v) aryloxy residues optionally chiral, having from 6 to 24 carbon atoms, wherein the aryloxy residues are optionally alkyl substituted;
(vi) benzyl;
(vii) alkaloyl residues optionally chiral, having from 2 to 13 carbon atoms, wherein the alkaloyl residues are optionally aryl substituted;
(viii) aroyl residues optionally chiral, having from 7 to 25 carbon atoms, wherein the aryloxy residues are optionally alkyl substituted; and
(ix) alkoxycarbonyl residues optionally chiral, having from 2 to 13 carbon atoms;
(x) aryloxycarbonyl residues optionally chiral, having from 7 to 25 carbon atoms;
(xi) tosyl;
(xii) silyl residues optionally chiral, having from 3 to 15 carbon atoms;
(xiii) silyloxy residues optionally chiral, having from 3 to 15 carbon atoms;

Ar denotes respectively unsubstituted or substituted phenyl, alkyl-aryl, alkoxy-aryl; and
wherein $NR_3R_4R_5$ represents an amine and $R_3$, $R_4$ and $R_5$ are independently selected from residues to form a chiral amine, wherein two or all three among $R_3$, $R_4$ and $R_5$ may together form a ring structure, provided that at least one of $R_3$, $R_4$ and $R_5$ or said ring structure contains a chiral center.

**16.** The compound of formula II according to claim 15, wherein Ar, $NR_1R_2$, and $NR_3R_4R_5$ are independently selected from the definitions as set forth in anyone of claims 6 to 8.

**17.** Use of a compound as defined in any of claims 15 or 16 in a process for the preparation of antidiabetic agents, in particular (*R*)-3-amino-1-[3-(trifluoromethyl)-5,6,7,8-tetrahydro[1,2,4]triazol[4,3-*a*]pyrazin-7-yl]-4-(2,4,5-trifluorphenyl)butan-1-one.

Europäisches Patentamt
European Patent Office
Office européen des brevets

# EUROPEAN SEARCH REPORT

Application Number
EP 12 15 0892

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 2 308 829 A1 (JIANGSU HENGRUI MEDICINE CO LTD [CN]) 13 April 2011 (2011-04-13)<br>* paragraph [[0020]] - paragraph [[0029]]; claims; examples *<br>----- | 1-13,15, 16 | INV.<br>C07C211/27<br>C07C231/06<br>C07C239/20<br>C07F5/02<br>C07C227/34<br>C07C269/06<br>C07C303/40<br>C07C67/31 |
| Y | EP 1 059 286 A1 (LILLY CO ELI [US]) 13 December 2000 (2000-12-13)<br>* page 3, line 17 - page 4, line 2; claims; example 2 *<br>----- | 1-13,15, 16 | |
| X | ZHU Y ET AL: "Synthesis, biological assay in vitro and molecular docking studies of new imidazopyrazinone derivatives as potential dipeptidyl peptidase IV inhibitors",<br>EUROPEAN JOURNAL OF MEDICINAL CHEMISTRY, EDITIONS SCIENTIFIQUE ELSEVIER, PARIS, FR,<br>vol. 45, no. 11,<br>1 November 2010 (2010-11-01), pages 4953-4962, XP027408906,<br>ISSN: 0223-5234<br>[retrieved on 2010-08-12]<br>* page 4954, scheme 2; page 4955, scheme 3 *<br>----- | 14,17 | TECHNICAL FIELDS SEARCHED (IPC)<br>C07C<br>C07F |
| A,D | WO 2011/102640 A2 (HANMI HOLDINGS CO LTD [KR]; CHANG JI-YEON [KR]; LEE HYUN SEUNG [KR]; K) 25 August 2011 (2011-08-25)<br>* claims; examples *<br>----- | 1-17 | |

1

The present search report has been drawn up for all claims

| Place of search | Date of completion of the search | Examiner |
|---|---|---|
| The Hague | 24 May 2012 | Zervas, Brigitte |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 12 15 0892

This annex lists the patent family members relating to the patent documents cited in the above-mentioned European search report.
The members are as contained in the European Patent Office EDP file on
The European Patent Office is in no way liable for these particulars which are merely given for the purpose of information.

24-05-2012

| Patent document cited in search report | Publication date | Patent family member(s) | Publication date |
|---|---|---|---|
| EP 2308829 A1 | 13-04-2011 | CA 2731245 A1 | 28-01-2010 |
| | | CN 101633625 A | 27-01-2010 |
| | | EP 2308829 A1 | 13-04-2011 |
| | | JP 2011528671 A | 24-11-2011 |
| | | KR 20110033276 A | 30-03-2011 |
| | | US 2011130587 A1 | 02-06-2011 |
| | | WO 2010009630 A1 | 28-01-2010 |
| EP 1059286 A1 | 13-12-2000 | CA 2310425 A1 | 09-12-2000 |
| | | EP 1059286 A1 | 13-12-2000 |
| | | JP 2000351740 A | 19-12-2000 |
| WO 2011102640 A2 | 25-08-2011 | KR 20110095672 A | 25-08-2011 |
| | | WO 2011102640 A2 | 25-08-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03004498 A
- WO 09064476 A
- WO 04085378 A
- WO 05097733 A
- WO 06081151 A
- WO 2011102640 A2


### Non-patent literature cited in the description


- **HANSEN K. B.** *Org. Process Res. Dev.,* 2005, vol. 9, 634-639
- **HANSEN, K. B.** *J. Am. Chem. Soc.,* 2009, vol. 131, 8798-8804
- **KIM, Y. ; PARK, J. ; KIM, M-J.** *ChemCatChem,* 2011, vol. 3, 271-277
- **PARVULESCU, A. N. ; JACOBS, P. A. ; DE VOS, D. E.** *Adv. Synth. Catal.,* 2008, 113-121
- **KIM, M-J. ; KIM, W-H. ; HAN, K. ; CHOI, Y-K. ; PARK, J.** *Org. Lett,* 2007, 91157-1159
- **PAMIES, O. ; BACKWALL, J. E.** *Chem. Rev.,* 2003, vol. 103, 3247-3262
- **LIU, F.** *J. Chem. Res.,* 2010, vol. 34, 230-232